# EUROPÄISCHE PATENTANMELDUNG

(11) **EP 0 773 703 A1**
(43) Veröffentlichungstag der Anmeldung: **14.05.1997**
(21) Anmeldenummer: 96117982.7
(22) Anmeldetag: 08.11.1996
(51) Int. Cl.: H05B 3/00, B01D 1/00, A61L 9/03, B60H 3/00

(54) **Elektrisches Gerät zum Verdampfen von flüssigen Aromastoffen in Automobilen**

(30) Priorität: 08.11.1995 AT 606/95
(71) Anmelder: Suritsch, Martin, 2301 Grossenzersdorf (AT)
(72) Erfinder: Suritsch, Martin, 2301 Grossenzersdorf (AT)
(74) Vertreter: KUHNEN, WACKER & PARTNER

(57) **Zusammenfassung**

Beschrieben wird ein elektrisches Gerät (10) zum Verdampfen von flüssigen Aromastoffen in Automobilen, das auf einfache Weise eine angenehme Aromanote im Automobil erzeugt. Das Gerät weist im wesentlichen einen Stecker (12) und einen pfeifenkopfähnlichen Teil (14) auf, in dem ein mit flüssigen Aromastoffen vorgefüllter, herausnehmbarer Becher (18) eingesetzt werden kann. Als Schutz gegen ein Herausspritzen von Aromaflüssigkeit ist ein Deckel (24) vorgesehen, der vorzugsweise den Becher im pfeifenkopfähnlichen Teil fixiert. Der Becher hat oberseitig eine dünne, wasserundurchlässige Haut (22), die leicht vor oder nach dem Einsetzen des Bechers abgezogen werden kann.

## Beschreibung

Die Erfindung betrifft ein elektrisches Gerät zum Verdampfen von flüssigen Aromastoffen in Automobilen, gemäß dem Oberbegriff des Anspruchs 1.

Es wurde bereits ein derartiges Gerät vorgeschlagen, das sich erstmalig zum Verdampfen von flüssigen Aromastoffen in Kraftfahrzeugen eignet. Mit einem solchen Gerät erweist es sich jedoch als ziemlich schwierig, Aromastoffe nachzufüllen. Denn die Aromaschale ist in der Regel sehr klein, so daß es während der Fahrt fast unmöglich und im Stillstand ebenfalls schwer ist, Flüssigkeit nachzuschütten ohne etwas zu vergießen. Dies führt aufgrund der hohen Aromakonzentration derartiger Stoffe zu unerwünschter, weil unkontrollierter Aromaentwicklung im Fahrgastraum. Es erweist sich bei dem vorgeschlagenen Gerät außerdem als nachteilig, daß hochspritzende Flüssigkeit nur bedingt von einem tropfenabfangenden Sieb aufgenommen werden kann, so daß es bei unruhiger Fahrt des Automobils auf unebenen Straßen ebenfalls zu einem unkontrollierten Austritt von Flüssigkeit kommen kann. Außerdem ist es mit dem vorgeschlagenen Gerät generell schwierig, die Reinheit des Aromas in der betreffenden und gewünschten Qualität und Aromanote über einen längeren Zeitraum aufrecht zu erhalten. Mit einer Innenbeschichtung der den Aromastoff aufnehmenden Vertiefung im pfeifenkopfähnlichen Teil kann diesbezüglich zwar eine Verbesserung erzielt werden, gleichzeitig verteuert diese Maßnahme aber das Gerät.

Es ist deshalb Aufgabe der Erfindung, eine Gerät gemäß dem Oberbegriff des Anspruchs 1 derart weiterzubilden, daß die vorher genannten Nachteile ausgeräumt werden können. Im einzelnen soll ein preisgünstiges Gerät geschaffen werden, mit dem es gelingt, ein individuell einstellbares Aroma im Fahrgastraum über einen beliebigen Zeitraum in nahezu unveränderter Qualität aufrecht zu erhalten.

Diese Aufgabe wird durch die Merkmale des Anspruchs 1 gelöst.

Erfindungsgemäß wird der pfeifenkopfähnliche Teil so umgestaltet, daß er den Aromastoff in der vorbestimmten Dosierung in einer auswechselbaren Kartusche aufnimmt. Dadurch wird nicht nur das Gerät preisgünstiger. Es wird darüber hinaus sichergestellt, daß nach dem Verbrauch der Aromaflüssigkeit problemlos eine Neubestückung des Geräts stattfinden kann, ohne daß man Gefahr läuft, daß Aromastoff unkontrolliert austritt. Dabei ergibt sich der zusätzliche Vorteil, daß Reinigungsarbeiten am Gerät praktisch entfallen können, selbst wenn zeitlich unmittelbar hintereinander vollkommen unterschiedliche Aromastoffe verwendet werden sollten. Der Geruch im Fahrgastraum ist demnach wesentlich feiner an die individuellen Wünsche der Insassen anpaßbar. Ein Nachfüllen der Kartusche ist entbehrlich, so daß die eingangs geschilderten Handhabungsprobleme entfallen.

Vorteilhafte Ausgestaltungen sind Gegenstand der Unteransprüche.

Mit der Weiterbildung des Anspruchs 2 ergibt sich der Vorteil, daß das Gerät in allen Fahrzeugtypen mit unterschiedlichster Orientierung der Buchse des Zigarettenanzünders verwendet werden kann, ohne die Kartusche schräg stellen zu müssen.

Die abziehbare Folie des Anspruchs 3 stellt sicher, daß der Aromastoff über einen langen Zeitraum unverfälscht bleibt, so daß er ohne Qualitätseinbußen gelagert werden kann. Gleichzeitig ist die Kartusche gegen Auslaufen von Aromastoff gesichert.

Wenn der die Kartusche bildende Becher gemäß Anspruch 4 mittels eines Deckels fixiert wird, ergibt sich eine bauliche Vereinfachung des Geräts, indem der Deckel eine Doppelfunktion erfüllt. Er sorgt für die Halterung der Kartusche und gleichzeitig dafür, daß nach oben spritzende Aromaflüssigkeit zuverlässig nach dem Prinzip einer Labyrinthabschirmung nach außen abgeschirmt wird.

Mit der Gestaltung des Anspruch 5 wird das Auswechseln der Kartusche weiter vereinfacht, da der Deckel unverlierbar am pfeifenkopfähnliche Teil über das Gelenk gehalten ist.

Die Gelenkausbildung des Anspruchs 6 hat herstellungstechnische und handhabungstechnische Vorteile.

Weil die auf den Deckel einwirkenden Kräfte klein sind, genügt zur Lagefixierung ein Schnappeingriff mit dem Einfassungsrand des pfeifenkopfähnliche Teils.

Es hat sich gezeigt, daß bereits sehr wenig Energie ausreicht, den Aromastoff in ausreichender Menge freizusetzen. Eine besonders vorteilhafte Heizquelle ist ein Glühlämpchen, da dies in Kraftfahrzeugen ohnehin in großem Umfang eingesetzt wird, so daß geringe Kosten entstehen. Auch für die Fassung können kostengünstige Normteile verwendet werden.

Mit dem Durchbruch des Anspruchs 9 ergibt sich eine gut sichtbare Funktionskontrolle des Geräts, wobei der Durchbruch gleichzeitig dazu genutzt werden kann, den Zugang zum Glühlämpchen zu Auswechselzwecken zu erleichtern.

Das Gelenk des Anspruchs 10 hat den Vorteil eines einfachen und kostengünstigen Aufbaus.

Die Aufnahme des Bechers gemäß Anspruch 11 sorgt dafür, daß der Becher ausschließlich -falls erwünscht- in einer vorbestimmten Ausrichtung im pfeifenkopfähnlichen Teil eingebaut werden kann.

Gemäß Anspruch 12 kann der Radialvorsprung in vorteilhafter Weise zum Greifen der Kartusche herangezogen werden. Vorteilhaft ist die Anordnung von zwei einander diametral gegenüberliegenden Radialvorsprüngen.

Die Gestaltung gemäß Anspruch 14 hat den Vorteil, daß die Kartusche wesentliche Teile der Spritzabschirmung bereits integriert. Der die Kartusche haltende Deckel kann somit vereinfacht werden.

Nachfolgend werden anhand schematischer Zeichnungen mehrere Ausführungsbeispiele der Erfindung näher erläutert. Es zeigen:
- Fig. 1: einen schematischen Längsschnitt des erfindungsgemäßen elektrischen Gerätes zum Verdampfen von flüssigen Aromastoffen;
- Fig. 2A: die Draufsicht des im Gerät verwendeten Deckels gemäß einer ersten Ausführungsform;
- Fig. 2B: den Schnitt IIA-IIA in Fig. 2A;
- Fig. 3: eine Schnittansicht des Bechers einer ersten Ausführungsform;
- Fig. 4A: die Draufsicht des Deckels entsprechend einer weiteren Ausführungsform der Erfindung;
- Fig. 4B: den Schnitt IVA-IVA in Fig. 4A;
- Fig. 5A: die Draufsicht des pfeifenkopfähnlichen Teils gemäß einer weiteren Ausführungsform der Erfindung;
- Fig. 5B: den Schnitt VA-VA in Figur 5A;
- Fig. 6A: eine Schnittansicht des Bechers entsprechend einer weiteren Ausführungsform; und
- Fig. 6B: die Draufsicht des Bechers gemäß Fig. 6B.

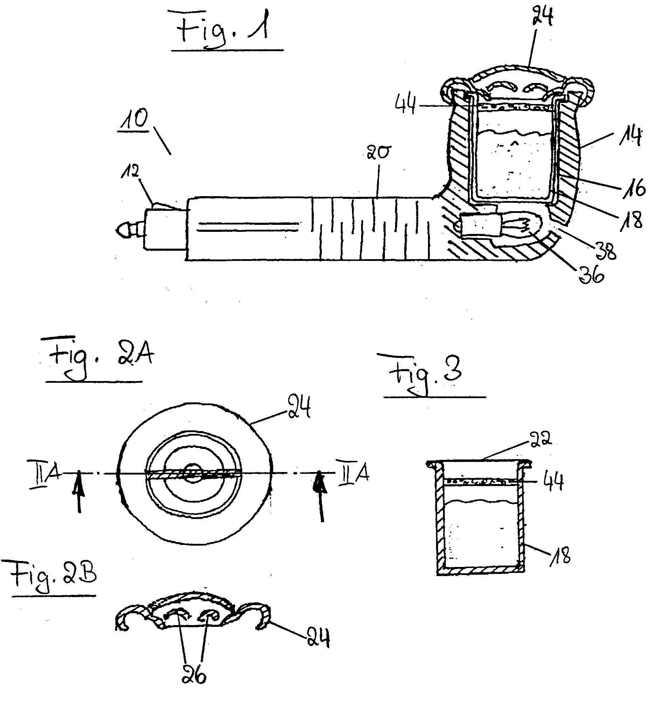

In Figur 1 ist mit dem Bezugszeichen (10) ein elektrisches Gerät zum Verdampfen von flüssigen Aromastoffen bezeichnet. Das elektrische Gerät (10) weist einen Stecker (12) zum Einstecken in die Buchse eines Zigarettenanzünders und einen pfeifenkopfähnlichen Teil (14) zur Aufnahme von Aromastoffen auf. Der Stecker (12) und der pfeifenkopfähnliche Teil (14) sind durch einen Hals (20) verbunden, der flexibel ist, sich leicht verbiegen läßt und dann in seiner Stellung verharrt, wobei er vorzugsweise in jeder Stellung fixierbar ist. In dem pfeifenkopfähnlichen Teil (14) befindet sich eine Aufnahme (16) für einen mit flüssigen Aromastoffen gefüllten, herausnehmbaren Becher (18). Der Becher (18) ist mit einer porösen Schicht oder einem Filtertuch (44) verschließbar, worüber vor Gebrauch eine leicht abziehbare, wasserundurchlässige Folie (22) gespannt ist. Weiterhin ist der Becher (18) im pfeifenkopfähnlichen Teil (14) vorzugsweise formschlüssig mit einem Deckel (24) fixierbar. In dem elektrischen Gerät (10) ist ein Glühlämpchen (36) zum Beheizen angeordnet. Im Bereich des Glühlämpchens (36) befindet sich ein Durchbruch (38) zu einer gut sichtbaren Funktionskontrolle des Gerätes, wobei gleichzeitig der Durchbruch (38) dazu genutzt werden kann, den Zugang zum Glühlämpchen (38) zu Auswechselzwecken zu erleichtern.

In den Figuren 2A und 2B ist jeweils eine Draufsicht und eine Schnittansicht bezüglich der Linie IIA einer ersten in Figur 1 dargestellten Ausführungsform des im Gerät (10) verwendeten Deckels (24) gezeigt. Zur Verhinderung des Herausspritzens von Aromaflüssigkeit besteht der Deckel (24) aus mehreren, sich überlappenden, im Querschnitt U-oder V- förmigen , nach unten konkav gebogenen Ringen oder Streben (26).

Figur 3 zeigt eine Schnittansicht des Bechers (18) einer ersten in Figur 1 beschriebenen Ausführungsform.

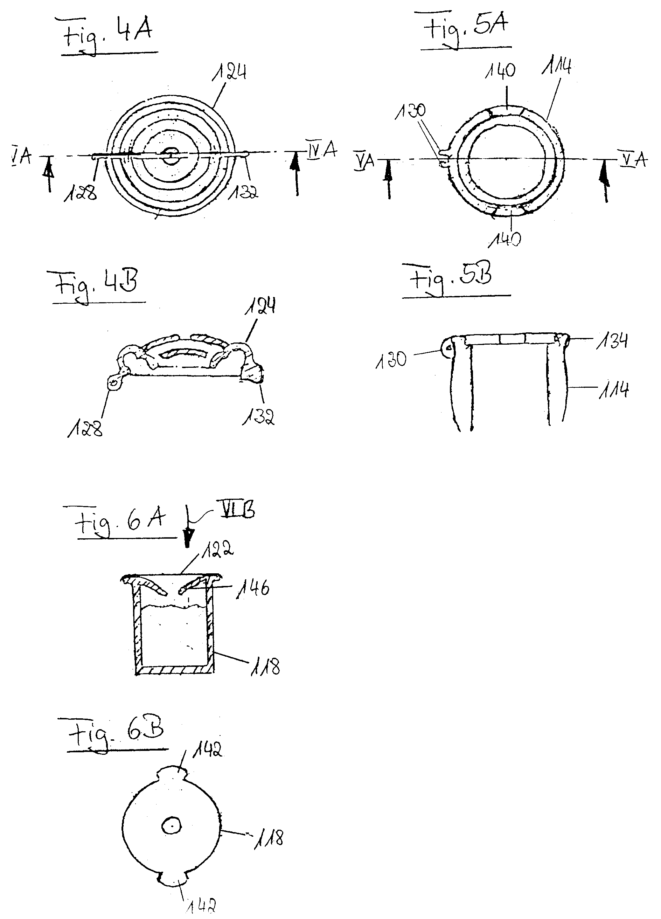

In den Figuren 4A und 4B ist jeweils eine Draufsicht und eine Schnittansicht bezüglich der Linie IVA einer zweiten Ausführungsform des verwendeten Deckels (124) gezeigt. Der Deckel (124) besitzt einen Fortsatz (128) und einen elastischen Clip (132).

In den Figuren 5A und 5B ist jeweils eine Draufsicht und eine Schnittansicht bezüglich der Linie VA einer zweiten Ausführungsform des pfeifenkopfähnlichen Teiles (114) dargestellt. Der pfeifenkopfähnliche Teil (114) weist zwei backenartige Fortsätze (130) auf, die zusammen mit dem Fortsatz (128) des Deckels (124) eine Schnappverbindung eingehen. Am oberen Fassungsrand (134) sind zwei Aussparungen (140) vorgesehen, die zur vorzugsweise passungsgenauen Aufnahme eines entsprechend geformten Bechers (118) geeignet sind.

Die Figuren 6A und 6B zeigen jeweils eine Schnittansicht und eine Draufsicht einer zweiten Ausführungsform des Bechers (118). Innenseitig im Bereich seines oberen Randes weist der Becher (118) eine trichterartige, einstückig an den Becher (118) geformte Abschirmungswand (146) auf. Desweiteren sind am oberen Rand Radialvorsprünge (142) vorgesehen, die vorzugsweise passungsgenau in die Aussparungen (140) des pfeifenkopfähnlichen Teiles (114) eingefügt werden können.

Mit dem erfindungsgemäßen Aufbau des elektrischen Gerätes zum Verdampfen von flüssigen Aromastoffen in Automobilen ist es nunmehr möglich, problemlos und ohne Reinigungsaufwand aromahaltige Kartuschen auch während der Fahrt auszuwechseln. Darüberhinaus ist sowohl eine verschmutzungsfreie Handhabung, als auch ein verschmutzungsfreier Betrieb gewährleistet.

Die Erfindung schafft somit ein elektrisches Gerät zum Verdampfen von flüssigen Aromastoffen in Automobilen, das auf einfache Weise eine angenehme Aromanote im Automobil erzeugt. Das Gerät weist im wesentlichen einen Stecker und einen pfeifenkopfähnlichen Teil auf, in dem ein mit flüssigen Aromastoffen vorgefüllter, herausnehmbarer Becher eingesetzt werden kann. Als Schutz gegen ein Herausspritzen von Aromaflüssigkeit ist ein Deckel vorgesehen, der vorzugsweise den Becher im pfeifenkopfähnlichen Teil fixiert. Der Becher hat oberseitig eine dünne, wasserundurchlässige Haut, die leicht vor oder nach dem Einsetzen des Bechers abgezogen werden kann.

## Patentansprüche

1. Elektrisches Gerät (10) zum Verdampfen von flüssigen Aromastoffen in Automobilen, mit einem Stecker (12) zum Einstecken in die Buchse eines Zigarettenanzünders und einem pfeifenkopfähnlichen Teil (14;114) zur Aufnahme des Aromastoffs, wobei in diesem eine Einrichtung zum Beheizen angeordnet ist, **dadurch gekennzeichnet, daß** der pfeifenkopfähnliche Teil (14;114) eine Aufnahme (16) für einen mit flüssigen Aromastoffen gefüllten, herausnehmbaren Becher (18;118) hat.

2. Gerät nach Anspruch 1, **dadurch gekennzeichnet, daß** der Stecker (12) und der pfeifenkopfähnliche Teil (14;114) durch einen Hals (20) verbunden sind, der flexibel ist, sich leicht verbiegen läßt und dann in seiner Stellung verharrt, wobei er vorzugsweise in jeder Stellung fixierbar ist.

3. Gerät nach Anspruch 1 oder 2, **dadurch gekennzeichnet, daß** über den Becher (18;118) vor Gebrauch eine leicht abziehbare, wasserundurchlässige Folie (22;122) gespannt ist.

4. Gerät nach einem der Ansprüche 1 bis 3, **dadurch gekennzeichnet, daß** der Becher (18;118) mittels eines Deckels (24;124) im pfeifenkopfähnlichen Teil (14;114) vorzugsweise formschlüssig fixierbar ist, welcher zur Verhinderung des Herausspritzens von Aromaflüssigkeit aus mehreren, sich überlappenden, im Querschnitt U- oder V-förmigen, nach unten konkav gebogenen Ringen oder Streben (26) besteht.

5. Gerät nach Anspruch 4, **dadurch gekennzeichnet, daß** der Deckel (124) einen Fortsatz (128) besitzt, der zusammen mit zwei weiteren backenartigen Fortsätzen (130) des pfeifenkopfähnlichen Teiles (114) zur Ausbildung eines Gelenks zusammensetzbar ist.

6. Gerät nach Anspruch 5, **dadurch gekennzeichnet, daß** der Fortsatz (128) des Deckels (124) mit den backenartigen Fortsätzen (130) eine Schnappverbindung eingeht.

7. Gerät nach einem der Ansprüche 4 bis 6, **dadurch gekennzeichnet, daß** der Deckel (124) zum Öffnen und Verschließen mit einem elastischen Clip (132) versehen ist, der in Schnappeingriff mit einem oberen Einfassungsrand (134) des pfeifenkopfähnlichen Teils (14;114) bringbar ist.

8. Gerät nach einem der Ansprüche 1 bis 7, **dadurch gekennzeichnet, daß** die Einrichtung zum Beheizen von einem Glühlämpchen (36) gebildet ist.

9. Gerät nach Anspruch 8, **dadurch gekennzeichnet, daß** der pfeifenkopfähnliche Teil (14) im Bereich des Glühlämpchens (36) einen Durchbruch (38) hat.

10. Gerät nach einem der Ansprüche 2 bis 9, **dadurch gekennzeichnet, daß** der Hals (20) ein Materialgelenk ausbildet.

11. Gerät nach einem der Ansprüche 1 bis 10, **dadurch gekennzeichnet, daß** der Becher (118) verdrehsicher in der Aufnahme (116) gehalten ist.

12. Gerät nach Anspruch 11, **dadurch gekennzeichnet, daß** der pfeifenkopfähnliche Teil (114) an seinem oberen Umfangsrand (134) zumindest eine Aussparung (140) hat, die zur vorzugsweise passungsgenauen Aufnahme eines entsprechenden Radialvorsprungs (142) am oberen Rand des Bechers (118) dient.

13. Gerät nach einem der Ansprüche 1 bis 12, **dadurch gekennzeichnet, daß** der Becher (18) mit einer porösen Schicht oder einem Filtertuch (44) verschließbar ist, worüber vor Gebrauch eine leicht abziehbare, wasserundurchlässige Folie (22) gespannt ist.

14. Gerät nach einem der Ansprüche 1 bis 12, **dadurch gekennzeichnet, daß** der Becher (118) innenseitig im Bereich seines oberen Randes eine trichterartige Abschirmungswand (146) hat.

15. Gerät nach Anspruch 14, **dadurch gekennzeichnet, daß** die Abschirmungswand (146) einstückig an den Becher (118) angeformt ist.
